# FASCICULE DE BREVET EUROPEEN

(11) **EP 3 104 897 B1**
(45) Date de publication et mention de la délivrance du brevet: **06.04.2022**
(21) Numéro de dépôt: 15708308.0
(22) Date de dépôt: 29.01.2015
(51) Int. Cl.: A61L 2/07, A23L 3/04, A23L 5/20

(54) **SYSTÈME DE TRAITEMENT THERMIQUE D'ARTICLES**
SYSTEM ZUR THERMISCHEN BEHANDLUNG VON GEGENSTÄNDEN
SYSTEM FOR THE HEAT TREATMENT OF ARTICLES

(30) Priorité: 29.01.2014 FR 1450712
(43) Date de publication de la demande: 21.12.2016
(73) Titulaire: Steriflow, 42300 Roanne (FR)
(72) Inventeur: ROUMAGNAC, Jean-Patrick, F-42300 Roanne (FR)
(74) Mandataire: Gevers & Orès
(86) Numéro de dépôt international: PCT/IB2015/050677
(87) Numéro de publication internationale: WO 2015/114556

(56) Documents cités:
- EP-A1- 1 462 156
- EP-A2- 0 138 688
- FR-A- 940 097
- FR-A- 1 465 121
- GB-A- 719 880
- US-A1- 2003 159 595

## Description

L'invention se rapporte à un système de traitement thermique d'articles, en particulier un autoclave ou un stérilisateur.

De nombreux produits alimentaires et pharmaceutiques doivent subir un traitement thermique (cuisson, pasteurisation, stérilisation) pour être vendus. Il s'agit, par exemple, de produits liquides tels que les produits lactés, les soupes, les purées, les jus de fruits, mais également de produits solides contenus dans des emballages divers tels que des boîtes de conserve.

Le traitement thermique doit pouvoir être fait de manière homogène sur une quantité industrielle d'articles. Par quantité industrielle, on entend un poids supérieur à 100 kilos et, plus précisément un poids d'une tonne et plus d'articles.

Plusieurs systèmes sont actuellement connus. La présente invention concerne les autoclaves horizontaux.

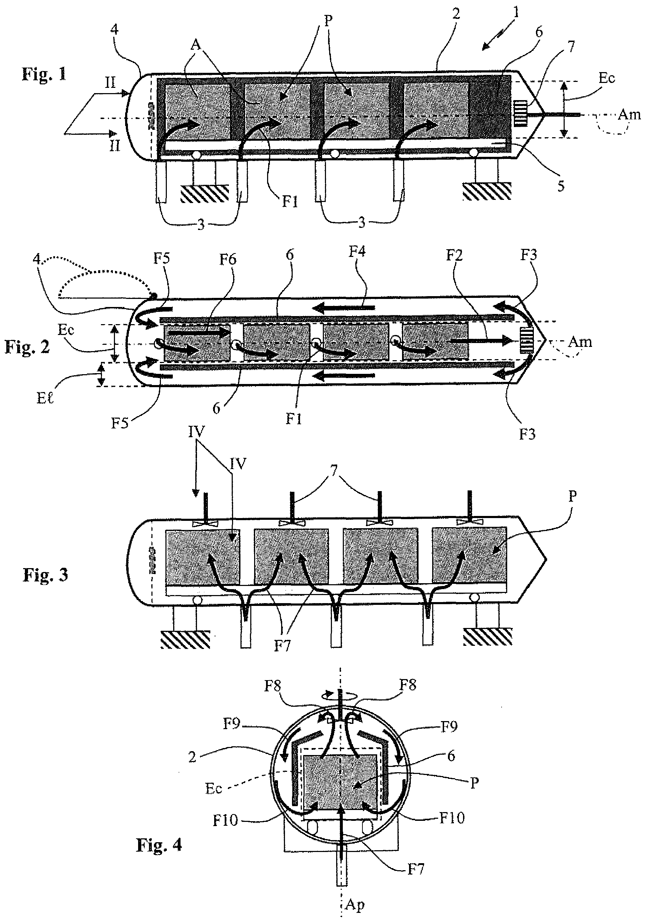

Un tel autoclave 1 connu est illustré aux figures 1 et 2.

Il comprend une enceinte oblongue 2, cylindrique ou parallélépipédique, présentant un axe longitudinal médian Am, l'enceinte étant étanche et apte à être mise sous pression. Cette enceinte comprend :
- au moins une entrée 3 d'injection du fluide caloporteur ;
- une porte d'entrée 4 agencée à une extrémité longitudinale de l'enceinte pour permettre l'insertion des articles A dans l'enceinte ; et
- un support 5 pour les articles, agencé le long de l'axe longitudinal médian, de sorte qu'en utilisation, les articles sont placés centralement dans l'enceinte, dans un espace central Ec le long de l'axe longitudinal médian. Un espace latéral Eℓ déterminé est ménagé entre l'enceinte et les articles.

Les articles A à traiter sont rangés dans des paniers P, lesquels sont introduits dans l'autoclave par la porte. La porte est ensuite fermée et le traitement thermique peut démarrer.

La vapeur est utilisée couramment comme fluide caloporteur pour chauffer les articles (cuisson, pasteurisation, stérilisation) alimentaires en particulier.

Lorsqu'elle est utilisée pure, c'est-à-dire en l'absence d'incondensables (air), le transfert de chaleur est efficace et homogène.

Lorsqu'elle est mélangée à de l'air, ce qui est systématiquement le cas dans un stérilisateur à surpression utilisé actuellement dans l'industrie, l'homogénéité du mélange ne peut être assurée que par un brassage efficace à l'intérieur de l'enceinte.

Ce brassage permet d'assurer qu'il n'y a pas de poches d'air et donc de zones froides dans lesquelles les articles (boîtes, bocaux, sachets, barquettes...) seraient sous stérilisés. Il permet également d'améliorer le coefficient d'échange sur les articles traités si la vitesse de circulation autour des articles est suffisante.

Pour améliorer la circulation interne du fluide caloporteur, l'enceinte 2 est équipée d'un habillage interne 6 constitué par des parois pleines séparant latéralement les articles de l'enceinte. Autrement dit, l'espace latéral Eℓ ménagé entre les articles A et l'enceinte 2 est délimité par l'enceinte 2 et l'habillage interne 6.

Différents systèmes utilisant ce procédé dit « à mélange d'air et de vapeur », existent.

Dans un premier type de stérilisateur illustré aux figures 1 et 2, un moyen de brassage 7 est agencé axialement sur l'axe longitudinal médian Am du stérilisateur horizontal. Le moyen de brassage est soit un ventilateur axial soit une turbine centrifuge (stérilisateur de marque STERlSTEAM^{®} de BARRIQUAND-STERIFLOW^{®}).

Cependant, l'expérience montre que ce type de stérilisateur ne donne pas entièrement satisfaction car la configuration limite, de fait, la taille du moyen de brassage et donc son efficacité. Il peut donc exister encore des poches plus froides, de sorte qu'il peut s'avérer nécessaire d'allonger la durée de traitement thermique et de brassage.

Par ailleurs, avec ce type de stérilisateur, la phase de refroidissement des produits après la phase de stérilisation, peut être faite selon deux procédés différents.

Un premier procédé consiste à faire circuler dans l'enceinte de l'eau rafraîchie par un échangeur de chaleur. Ce procédé est très efficace en termes de rapidité du refroidissement, mais implique un mouillage important des produits.

Un deuxième procédé, dit « sec », consiste à injecter dans l'enceinte de l'air refroidi par des batteries froides. Cependant cette injection d'air froid entraine la condensation de la vapeur présente dans l'enceinte sur les produits, ce qui les mouille légèrement.

Il est donc systématiquement nécessaire de laisser reposer les produits après la stérilisation afin de laisser l'eau s'évaporer, ce qui rallonge la durée globale du traitement. Le moyen de brassage classiquement utilisé, du fait de ses dimensions obligatoirement réduites, ne peut pas servir à accélérer la phase de refroidissement.

Un objectif de la présente invention est de raccourcir la durée du traitement, notamment en raccourcissant la durée de la phase de refroidissement. L'intérêt d'une vitesse de circulation plus élevée est primordial.

Afin de tenter de résoudre les problèmes posés par ce type de stérilisateur, il a été proposé un second type de stérilisateur, illustré aux figures 3 et 4, dans lequel le moyen de brassage 7 est disposé radialement, de manière à générer une circulation radiale du fluide caloporteur, panier par panier. Ce faisant, il a été nécessaire de prévoir au moins autant de moyens de brassage 7 que de paniers P à stériliser.

Cependant, si la taille de chaque moyen de brassage est plus petite que dans le premier type de stérilisateur, leur nombre est multiplié, ce qui augmente le coût et la complexité de l'équipement (maintenance des moteurs, maintenance des systèmes d'étanchéité des arbres d'entraînement, etc.).

En outre, les systèmes radiaux nécessitent un habillage interne plus compliqué pour la création des veines gazeuses de chaque panier.

GB719 880, EP 0 138 688, EP 1 462 156, FR 1 465 121, FR 940 097 et US 2003/015595 divulguent tous un système de traitement thermique d'articles.

L'objectif de la présente invention est donc de proposer un système fiable de traitement thermique de articles en quantité industrielle (de 100 kg jusqu'à 10 tonnes, voire plus), permettant un meilleur traitement thermique à dépense d'énergie égale, plus économe tout en permettant une cadence de traitement élevée.

A cette fin, l'invention a pour objet un système de traitement thermique d'articles comme défini dans les revendications.

Selon d'autres modes de réalisation :
- le moyen de brassage peut être une turbine centrifuge ;
- la turbine centrifuge peut être munie d'une volute d'éjection du fluide caloporteur brassé par la turbine en utilisation ;
- le système peut comprendre deux moyens de brassage agencés latéralement par rapport à l'axe longitudinal médian, dans l'espace latéral situé entre l'enceinte et l'espace central, et de part et d'autre de l'espace central ;
- le support peut être un tablier monté coulissant par rapport à l'enceinte ;
- l'entrée d'injection d'un fluide caloporteur est destinée à l'injection d'un mélange air/vapeur d'eau ;
- l'enceinte peut comprendre, en outre, une porte de sortie agencée à une deuxième extrémité longitudinale de l'enceinte, à l'opposé de la porte d'entrée par rapport à l'espace central ;
- le système peut comprendre au moins un panier de stockage des articles ; et/ou
- le système peut comprendre au moins une sortie d'évacuation du fluide caloporteur.

D'autres caractéristiques de l'invention seront énoncées dans la description détaillée ci-après, faite en référence aux figures annexées qui représentent, respectivement :
- la figure 1, une vue schématique en coupe sagittale d'un premier mode de réalisation d'un système de traitement thermique selon l'état de la technique ;
- la figure 2, une vue schématique en coupe transverse, vue de dessus, selon la ligne II-II, du système de traitement thermique de la figure 1 ;
- la figure 3, une vue schématique en coupe sagittale d'un deuxième mode de réalisation d'un système de traitement thermique selon l'état de la technique ;
- la figure 4, une vue schématique en coupe frontale, selon la ligne IV-IV, du système de traitement thermique de la figure 3 ;
- la figure 5, une vue schématique en coupe sagittale d'un premier mode de réalisation d'un système de traitement thermique selon l'invention ;
- la figure 6, une vue schématique en coupe transverse, vue de dessus, selon la ligne VI-VI, du système de traitement thermique de la figure 5 ;
- la figure 7, une vue schématique en coupe transverse, vue de dessus, d'un deuxième mode de réalisation d'un système de traitement thermique selon l'invention ; et
- la figure 8, une vue schématique en coupe frontale, selon la ligne VIII-VIII, du système de traitement thermique de la figure 7.

La vapeur est utilisée couramment comme fluide caloporteur pour chauffer les articles (cuisson, pasteurisation, stérilisation) alimentaires en particulier.

Elle est injectée directement dans l'enceinte. De préférence, l'enceinte 2 comprend plusieurs entrées 3 d'injection de vapeur, avantageusement autant d'entrée que de paniers P, voire plusieurs entrées d'injections de vapeur par panier. L'injection se fait alors par le côté de chaque panier, latéralement, par le dessus et/ou le dessous.

Classiquement, ces entrées sont constituées par des tubulures secondaires d'une tubulure principale courant à l'intérieur ou à l'extérieur de l'enceinte.

Comme le montrent les figures 1 à 4, le principe général du brassage, connu des stérilisateurs de l'état de la technique, consiste à agencer un moyen de brassage à l'opposé d'une entrée de fluide caloporteur par rapport au(x) panier(s), c'est-à-dire par rapport à l'espace central Ec. Puis il est mis en œuvre de telle sorte qu'il aspire le fluide caloporteur depuis l'espace central où se trouvent les paniers P de manière à favoriser le flux de fluide caloporteur depuis l'entrée d'injection à travers les ou les paniers. Autrement dit, le fluide n'est pas pulsé vers l'espace central par le moyen de brassage, mais il est aspiré hors de l'espace central.

Le flux de fluide caloporteur est alors évacué par le moyen de brassage 7 depuis l'espace central Ec où se trouvent les paniers P, vers l'espace latéral Eℓ ménagé entre l'habillage interne et l'enceinte. Le fluide caloporteur est ensuite ré aspiré en boucle.

L'inventeur s'est aperçu que plusieurs problèmes posés par le premier type de stérilisateur (figures 1 et 2) ne sont pas résolus par les stérilisateurs à brassage radial (figures 3 et 4) en raison du fait qu'ils mettent en œuvre un même principe général de fonctionnement selon lequel le moyen de brassage, la ou les entrée(s) de fluide caloporteur et les paniers sont tous alignés par rapport à l'espace central Ec : dans le premier type de stérilisateur ils sont agencés le long de l'axe longitudinal médian (voir figure 2), et dans le second type de stérilisateur, ifs sont agencés le long d'un axe Ap perpendiculaire à l'axe longitudinal médian Am (voir figure 4).

Or, il est classiquement considéré que c'est cet alignement par rapport à l'espace central Ec qui permet un brassage optimal du fluide caloporteur du fait que l'aspiration est sans perte de charge, donc maximale, entre l'entrée de fluide caloporteur et le moyen de brassage.

Ainsi, le schéma de circulation du mélange air/vapeur pour le premier type de stérilisateur décrit en introduction est illustré dans les figures 1 et 2. La circulation est axiale, c'est-à-dire parallèlement à l'axe longitudinal médian Am. Après avoir été introduit dans l'espace central Ec selon les flèches F1 depuis la ou les entrées 3, le fluide caloporteur circule dans l'espace central Ec selon la flèche F2, puis il est aspiré par le moyen de brassage 7. Le fluide caloporteur diverge alors à partir du moyen de brassage. La circulation du fluide caloporteur à partir du moyen de brassage se fait alors radialement, vers les parois de l'enceinte, selon les flèches F3, puis longitudinalement dans l'espace latéral ménagé entre l'enceinte et les articles selon les flèches F4. Ensuite, à l'extrémité opposée de l'enceinte, le fluide recircule radialement selon les flèches F5 et converge vers le centre de l'enceinte puis longitudinalement selon la flèche F6 vers le moyen de brassage qui ré aspire le fluide et le remet en circulation.

Le schéma de circulation du mélange air/vapeur pour le deuxième type de stérilisateur décrit en introduction est illustré dans les figures 3 et 4. La circulation est radiale, sur la hauteur du stérilisateur horizontal.

Après avoir été introduit dans l'espace central selon les flèches F7 depuis la ou les entrées de fluide caloporteur, ce dernier circule radialement dans l'espace central, puis il est aspiré par le moyen de brassage. Le fluide caloporteur diverge alors à partir du moyen de brassage selon les flèches F8, puis circule le long des parois de l'enceinte dans l'espace latéral ménagé entre l'enceinte et les articles, selon les flèches F9. Ensuite, à l'extrémité opposée de l'enceinte, le fluide converge selon les flèches F10 vers l'espace central de l'enceinte puis le moyen de brassage qui ré aspire le fluide et le remet en circulation.

Compte-tenu des pertes de charges engendrées par les changements de directions et la longueur de l'enceinte, le rendement du moyen de brassage est faible dans le premier type de stérilisateur. Pour assurer un brassage suffisant, le moyen de brassage devrait être surdimensionné et serait donc encombrant. Pour pouvoir placer un tel moyen de brassage (turbine ou ventilateur) dans l'enceinte, il faudrait soit concevoir des stérilisateurs plus longs, sans augmentation du volume utile de stérilisation, soit réduire le nombre de paniers dans le stérilisateur pour laisser de la place au moyen de brassage.

L'alternative illustrée aux figures 3 et 4 n'est pas totalement satisfaisante car si la longueur du circuit de fluide est moins importante (uniquement sur la hauteur du stérilisateur), elle nécessite un habillage interne complexe.

En outre, comme avec le premier type de stérilisateur, les pertes de charge au travers des paniers varient selon le plan de chargement des articles (la compacité des emballages, le type de paniers ou de plateaux empilés, leur écartement, les circuits parasites...). Donc plus le stérilisateur est gros, plus le ou les moyens de brassage doivent être gros et plus le moteur qui les entraîne doit être puissant. Outre les coûts engendrés, il se pose des limites techniques, ainsi que des problèmes de vibration incompatibles avec une étanchéité durable du stérilisateur.

Des limites dimensionnelles et techniques s'imposent donc, de sorte qu'il n'est pas possible de concevoir des stérilisateurs économiquement viables de grande taille à partir des solutions connues.

L'inventeur s'est aperçu qu'en allant à l'encontre du préjugé concernant l'alignement des entrées de fluide et du moyen de brassage par rapport à la zone centrale, et en plaçant le moyen de brassage dans l'espace latéral ménagé entre l'espace central et l'enceinte, le brassage obtenu pouvait être de bien meilleure qualité.

Pour cela, l'invention propose de placer le moyen de brassage (de préférence une turbine) dans l'espace ménagé entre les articles et l'enceinte, de manière à brasser le fluide caloporteur et le faire circuler dans une direction longitudinale. Cet agencement permet l'utilisation de volutes permettant de concentrer, d'accélérer et de diriger le fluide caloporteur directement vers l'extrémité en regard du moyen de brassage, améliorant la circulation du fluide caloporteur dans l'enceinte et, par voie de conséquence, l'homogénéité du traitement thermique des articles.

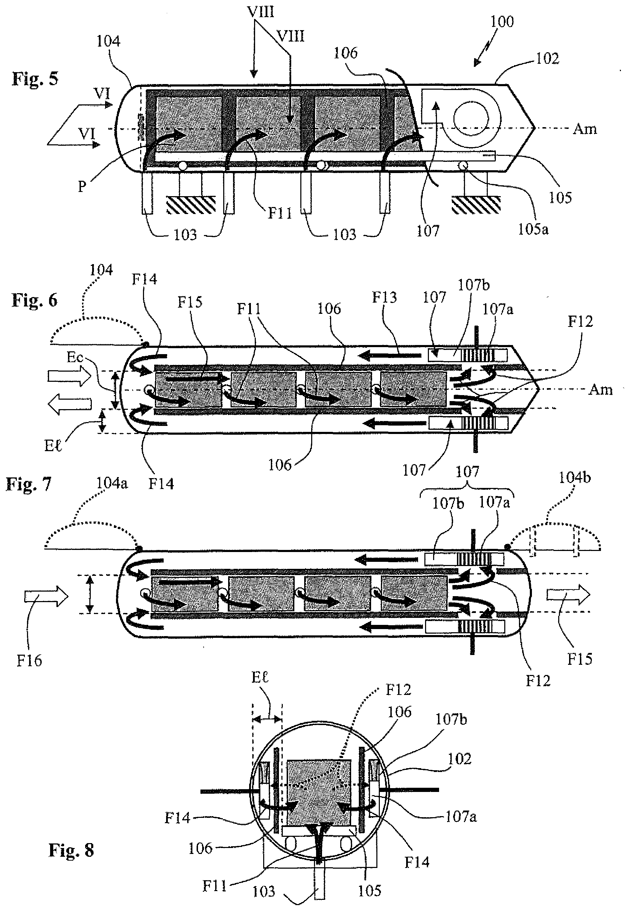

Un exemple de réalisation de l'invention est illustré à la figure 5.

Le système 100 de traitement thermique d'articles selon l'invention comprenant une enceinte oblongue 102 présentant un axe longitudinal médian Am, l'enceinte étant étanche et apte à être mise sous pression. Elle comprend:
- au moins une entrée d'injection d'un fluide caloporteur 103 ;
- une porte 104 d'entrée agencée à une extrémité longitudinale de l'enceinte 102 pour permettre l'insertion des articles dans l'enceinte ; et
- un support 105 agencé le long de l'axe longitudinal médian, de sorte qu'en utilisation, les articles sont placés à l'intérieur d'un espace central Ec dans l'enceinte, le long de l'axe longitudinal médian, un espace latéral déterminé Eℓ étant ménagé entre l'enceinte et l'espace central.

De préférence, les articles sont placés dans des paniers de stockage P, et l'enceinte comprend autant d'entrées 103 de fluide caloporteur que de paniers.

Selon l'invention, le système comprend au moins un moyen de brassage 107 du fluide caloporteur agencé latéralement par rapport à l'espace central Ec, dans l'espace latéral Eℓ ménagé entre l'enceinte et l'espace central, et orienté de manière à brasser et à faire circuler le fluide caloporteur dans une direction longitudinale le long de l'espace latéral.

Au contraire, dans l'état de la technique, le moyen de brassage est soit placé dans l'espace central (figures 1 et 2), soit placé latéralement, mais orienté de sorte que le fluide caloporteur est brassé radialement (figures 3 et 4).

L'enceinte comprend, en outre, au moins une paroi pleine 106 agencée entre le moyen de brassage et l'espace central, de sorte qu'en utilisation, le fluide caloporteur brassé par le moyen de brassage soit canalisé vers une extrémité de l'enceinte en circulant longitudinalement avant de revenir vers les articles. Ces parois pleines sont avantageusement des tôles constituant ainsi deux veines gazeuses latérales au travers desquelles le mélange circule.

La figure 6 illustre deux parois pleines 106 disposées longitudinalement ou encore parallèlement à l'axe Am. Elles sont plus particulièrement disposées de part et d'autre de l'espace central Ec ou encore du support 105.

Avantageusement, le système comprend deux moyens de brassage agencés latéralement par rapport à l'espace central, dans l'espace latéral déterminé entre l'enceinte et l'espace central, et de part et d'autre du support 105.

Ainsi, le schéma de circulation du mélange air/vapeur pour le stérilisateur selon l'invention est décrit en rapport avec la figure 6.

Après avoir été introduit dans l'espace central Ec selon les flèches F11 depuis la ou les entrées 103, le fluide caloporteur circule dans l'espace central Ec, puis il est aspiré par les moyens de brassage 107 situés latéralement. Le fluide caloporteur diverge alors avant les moyens de brassage selon les flèches F12. Autrement dit, la circulation du fluide caloporteur avant les moyens de brassage se fait radialement, vers les parois de l'enceinte, selon les flèches F12, puis, à partir des moyens de brassage 107, longitudinalement dans l'espace latéral ménagé entre l'enceinte et l'espace central Ec selon les flèches F13. Ensuite, à l'extrémité opposée de l'enceinte, le fluide recircule radialement selon les flèches F14 et converge vers le centre de l'enceinte puis longitudinalement selon la flèche F15 dans l'espace central Ec. Les moyen de brassage le remettent ensuite en circulation jusqu'à la fin du traitement.

Dans la suite de la description, le fluide caloporteur est un mélange air/vapeur d'eau, mais d'autres fluides caloporteurs peuvent êtres utilisés, du moment qu'ils peuvent être brassés par le moyen de brassage.

Le moyen de brassage étant situé dans l'espace latéral, il est possible de mettre en œuvre des ventilations plus conséquentes dans le même encombrement interne du stérilisateur, ou bien, à performance identique, de réduire leur taille.

De façon générale, le moyen de brassage permet d'aspirer le fluide caloporteur et de l'expulser dans une direction longitudinale à l'intérieur de l'enceinte.

Alternativement ou en combinaison, grâce à la place libre dans l'espace latéral Eℓ, il est possible de munir le moyen de brassage d'une volute et/ou d'un habillage, pour canaliser le flux de mélange air/vapeur entrant dans le moyen de brassage et/ou sortant du moyen de brassage.

De préférence, chaque moyen de brassage est une turbine centrifuge 107a, telle qu'une turbine à action à pales. En effet, une telle turbine aspire le mélange air/vapeur par son centre et l'expulse latéralement. Ce type de turbine est donc particulièrement bien adapté à l'agencement selon l'invention car il n'y a pas de perte de charge à l'expulsion hors de la turbine, contrairement aux agencements de l'état de la technique.

Cependant, installée telles quelles dans le stérilisateur, avec uniquement des tôles d'habillage interne 106 pour canaliser le flux, le rendement des turbines est voisin de 50 %.

Dans les stérilisateurs connus, tels qu'illustrés aux figures 1 à 4, il est possible d'utiliser des turbines centrifuge comme moyen de brassage. Néanmoins, il est impossible de munir ces turbines de volutes en raison de l'encombrement de ces volutes et de l'emplacement des moyens de brassage par rapport aux paniers (agencement axial-central ou radial).

Grâce au placement latéral des turbines 107a, par rapport à l'espace central Ec, il est possible d'équiper les turbines de volutes 107b.

Le mode de réalisation illustré aux figures 5 à 8 comprend avantageusement deux turbines centrifuges 107a munies chacune d'une volute d'éjection 107b permettant d'accélérer et de guider le flux de mélange air/vapeur sortant de la turbine. Le rendement global obtenu peut atteindre 75%.

Donc à puissance identique par rapport aux stérilisateurs de l'état de la technique, le dispositif permet d'augmenter sensiblement le débit, donc le brassage ainsi que les vitesses de circulation.

De façon préférée, un moyen de brassage comprend donc un pavillon d'aspiration central, une turbine centrifuge à réaction (aspirante) et une volute d'éjection.

En outre, l'utilisation de turbines centrifuges est particulièrement intéressante car les arbres d'entrainement 107c sont disposés perpendiculairement à l'axe longitudinal médian. Grâce au placement latéral, les turbines sont au plus proche de l'enceinte de sorte que les longueurs d'arbre nécessaires sont inférieures à la longueur nécessaire lorsque la turbine est placée axialement. Grâce à ces longueurs d'arbre inférieures, le système selon l'invention subit beaucoup moins de vibrations.

Également grâce au placement latéral des moyens de brassage 107 par rapport à l'espace central Ec, chaque moyen de brassage 107 est positionné hors de l'emplacement occupé par le train de paniers remplis d'articles.

Ainsi, comme illustré à la figure 7, la solution technique selon l'invention permet de prévoir deux portes 104a et 104b dans le stérilisateur, car les moyens de brassage ne se trouvent plus le long de l'axe longitudinal médian, ce qui libère le passage pour les paniers.

Ainsi, grâce à l'invention, l'enceinte peut comprendre une porte de sortie 104b agencée à une deuxième extrémité longitudinale de l'enceinte 102, à l'opposé de la porte d'entrée 104a par rapport à l'espace central Ec et au support 105.

La présence des deux portes opposées permet une cadence de stérilisation importante : pendant que les paniers stérilisés sortent par la porte de sortie 104b dans le sens de la flèche F15, des paniers à stériliser entrent par la porte d'entrée 104a dans le sens de la flèche F16.

Au contraire, avec un stérilisateur ne comprenant qu'une porte 104, l'entrée selon la flèche F17 et la sortie selon la flèche F18 se font par la même porte (voir figure 6).

Afin de faciliter l'entrée et la sortie des paniers, le support 105 pour les articles (ou les paniers comprenant les articles) est avantageusement un tablier monté coulissant par rapport à l'enceinte 102. Le système de coulissement peut être des patins ou des roulettes 105a.

Lorsque le traitement thermique est terminé, l'enceinte est toujours sous pression du fluide caloporteur. Avant d'ouvrir la ou les portes, il convient de dépressuriser l'enceinte en évacuant du fluide caloporteur. A cette fin, l'enceinte comprend de préférence au moins une sortie (non illustrée) d'évacuation du fluide caloporteur.

L'invention permet de mettre en œuvre des débits de brassage (circulation gazeuse) nettement plus élevés qu'avec les solutions traditionnelles. Elle permet donc d'améliorer l'homogénéité de température dans toute l'enceinte et de favoriser le transfert thermique.

## Revendications

1. Système (100) de traitement thermique d'articles (A) comprenant :
- une enceinte oblongue (102) présentant un axe longitudinal médian (Am), l'enceinte étant étanche et apte à être mise sous pression, et comprenant :
• au moins une entrée (103) d'injection d'un fluide caloporteur ;
• une porte d'entrée (104-104a) agencée à une extrémité longitudinale de l'enceinte pour permettre l'insertion des articles dans l'enceinte ;
- un support (105) pour les articles, apte à être agencé le long de l'axe longitudinal médian, de sorte qu'en utilisation, les articles sont placés à l'intérieur d'un espace central (Ec) dans l'enceinte, le long de l'axe longitudinal médian, un espace latéral déterminé (Eℓ) étant ménagé entre l'enceinte (102) et l'espace central (Ec) ;
- au moins un moyen de brassage (107, 107a-107b) du fluide caloporteur agencé latéralement par rapport à l'espace central (Ec), dans l'espace latéral (Eℓ) situé entre l'enceinte (102) et l'espace central (Ec), le moyen de brassage permettant d'aspirer le fluide caloporteur et de l'expulser dans une direction longitudinale à l'intérieur de l'enceinte (102), pour assurer la circulation du fluide caloporteur dans une direction longitudinale parallèle à l'axe longitudinal médian (Am) le long de l'espace latéral (Eℓ)
**caractérisé en ce qu'**au moins une paroi pleine (106) est agencée longitudinalement entre le moyen de brassage et l'espace central, de sorte qu'en utilisation, le fluide caloporteur brassé par le moyen de brassage soit canalisé vers une extrémité de l'enceinte avant de revenir vers les articles.

2. Système selon l'une quelconque des revendications 1, dans lequel le moyen de brassage est une turbine centrifuge (107a).

3. Système selon la revendication 2, dans lequel la turbine centrifuge (107a) est munie d'une volute (107b) d'éjection du fluide caloporteur brassé par la turbine en utilisation.

4. Système selon l'une quelconque des revendications 1 à 3, comprenant deux moyens de brassage (107, 107a-107b) agencés latéralement par rapport à l'axe longitudinal médian, dans l'espace latéral (Eℓ) situé entre l'enceinte (102) et l'espace central (Ec), et de part et d'autre de l'espace central (Ec).

5. Système selon l'une quelconque des revendications 1 à 4, dans lequel le support (105) est un tablier monté coulissant par rapport à l'enceinte (102).

6. Système selon l'une quelconque des revendications 1 à 5, dans lequel ladite au moins une entrée (103) est destinée à l'injection d'un mélange air/vapeur d'eau.

7. Système selon l'une quelconque des revendications 1 à 6, dans lequel l'enceinte comprend, en outre, une porte de sortie (104b) agencée à une deuxième extrémité longitudinale de l'enceinte, à l'opposé de la porte d'entrée (104a) par rapport à l'espace central (Ec).

8. Système selon l'une quelconque des revendications 1 à 7, comprenant au moins un panier de stockage (P) des articles (A).

9. Système selon l'une quelconque des revendications 1 à 8, comprenant au moins une sortie d'évacuation du fluide caloporteur.

## Patentansprüche

1. System (100) zur thermischen Behandlung von Artikeln (A), umfassend:
- eine längliche Einschließung (102), die eine mittlere Längsachse (Am) aufweist, wobei die Einschließung dicht und in der Lage ist, mit Druck beaufschlagt zu werden, und umfassend:
- mindestens einen Einlass (103) zur Injektion eines Wärmeträgerfluids;
- eine Einlasstür (104-104a), angeordnet an einem Längsende der Einschließung, um die Insertion des Artikels in die Einschließung zu erlauben;
- einen Träger (105) für die Artikel, der in der Lage ist, entlang der mittleren Längsachse derart angeordnet zu werden, dass bei Nutzung die Artikel im Inneren eines zentralen Raums (Ec) in der Einschließung entlang der mittleren Längsachse platziert werden, wobei ein festgelegter seitlicher Raum (Eℓ) zwischen der Einschließung (102) und dem zentralen Raum (Ec) ausgebildet wird;
- mindestens ein Mittel zum Durchmischen (107,107a-107b) des Wärmeträgerfluids, bezüglich des zentralen Raums (Ec) in dem seitlichen Raum (Eℓ) seitlich angeordnet, der sich zwischen der Einschließung (102) und dem zentralen Raum (Ec) befindet, wobei das Mittel zum Durchmischen erlaubt, das Wärmeträgerfluid anzusaugen und es in einer Längsrichtung im Inneren der Einschließung (102) auszustoßen, um die Zirkulation des Wärmeträgerfluids in einer Längsrichtung parallel zu der mittleren Längsachse (Am) entlang des seitlichen Raums (Eℓ) sicherzustellen
**dadurch gekennzeichnet, dass** mindestens eine feste Wand (106) zwischen dem Mittel zum Durchmischen und dem zentralen Raum derart längsgerichtet angeordnet ist, dass bei Nutzung das durch das Mittel zum Durchmischen durchmischte Wärmeträgerfluid in Richtung eines Endes der Einschließung kanalisiert wird, bevor es in Richtung der Artikel zurückkehrt.

2. System nach einem der Ansprüche 1, wobei das Mittel zum Durchmischen eine Zentrifugalturbine (107a) ist.

3. System nach Anspruch 2, wobei die Zentrifugalturbine (107a) mit einem Spiralgehäuse (107b) zum Ausstoßen von durch die Turbine bei Nutzung durchmischtem Wärmeträgerfluid ausgestattet ist.

4. System nach einem der Ansprüche 1 bis 3, umfassend zwei Mittel zum Durchmischen (107, 107a-107b), bezüglich der mittleren Längsachse in dem seitlichen Raum (Eℓ) seitlich angeordnet, der sich zwischen der Einschließung (102) und dem zentralen Raum (Ec) und beiderseits des zentralen Raums (Ec) befindet.

5. System nach einem der Ansprüche 1 bis 4, wobei der Träger (105) eine bezüglich der Einschließung (102) verschiebbar montierte Spritzwand ist.

6. System nach einem der Ansprüche 1 bis 5, wobei der mindestens eine Einlass (103) zur Injektion eines Luft/Wasserdampf-Gemisches bestimmt ist.

7. System nach einem der Ansprüche 1 bis 6, wobei die Einschließung des Weiteren eine Auslasstür (104b) umfasst, an einem zweiten Längsende der Einschließung der Einlasstür (104a) bezüglich des zentralen Raums (Ec) gegenüberliegend angeordnet.

8. System nach einem der Ansprüche 1 bis 7, umfassend mindestens einen Lagerkorb (P) der Artikel (A).

9. System nach einem der Ansprüche 1 bis 8, umfassend mindestens einen Auslass zur Evakuierung des Wärmeträgerfluids.

## Claims

1. A system (100) for the heat treatment of articles (A), comprising:
- an oblong enclosure (102) having a median longitudinal axis (Am), the enclosure being sealed and able to be pressurized, and comprising:
at least one injection inlet (103) for a heat transfer fluid;
an inlet door (104-104a) arranged at one longitudinal end of the enclosure to allow the insertion of the articles in the enclosure;
- a support (105) for the articles, able to be arranged along the median longitudinal axis, such that during use, the articles are placed inside a central space (Ec) in the enclosure, along the median longitudinal axis, a determined lateral space (Eℓ) being arranged between the enclosure (102) and the central space (Ec);
- at least one means for mixing (107, 107a-107b) the heat transfer fluid arranged laterally relative to the central space (Ec), in the lateral space (Eℓ) situated between the enclosure (102) and the central space (Ec), the mixing means allowing to aspire the heat transfer fluid and to expulse it into a longitudinal direction inside the enclosure (102), to ensure the circulation of the heat transfer fluid in a longitudinal direction parallel to the median longitudinal axis (Am) along the lateral space (Eℓ),
**characterized in that** at least one solid wall (106) arranged longitudinally between the mixing means and the central space, such that during use, the heat transfer fluid mixed by the mixing means is channeled toward one end of the enclosure before returning back toward the articles.

2. The system according to claim 1, wherein the mixing means is a centrifugal turbine (107a).

3. The system according to claim 2, wherein the centrifugal turbine (107a) is provided with an ejection volute (107b) for the heat transfer fluid mixed by the turbine during use.

4. The system according to one of the claims 1 to 3, comprising two mixing means (107, 107a-107b) arranged laterally relative to the median longitudinal axis, in the lateral space (Eℓ) situated between the enclosure (102) and the central space (Ec), and on either side of the central space (Ec).

5. The system according to one of claims 1 to 4, wherein the support (105) is an apron mounted sliding relative to the enclosure (102).

6. The system according to one of claims 1 to 5, wherein said at least one injection inlet (103) is intended to inject an air/steam mixture.

7. The system according to one of claims 1 to 6, wherein the enclosure further comprises an outlet door (104b) arranged at a second longitudinal end of the enclosure, opposite the inlet door (104a) relative to the central space (Ec).

8. The system according to one of claims 1 to 7, comprising at least one storage basket (P) for the articles (A).

9. The system according to one of claims 1 to 8, comprising at least one discharge outlet for the heat transfer fluid.
